# EUROPEAN PATENT SPECIFICATION

(11) **EP 2 825 149 B1**
(45) Date of publication and mention of the grant of the patent: **10.05.2017**
(21) Application number: 13761481.4
(22) Date of filing: 14.03.2013
(51) Int. Cl.: A61H 19/00, A61H 23/02, A61N 5/06, A61H 7/00, A61H 23/00

(54) **SEXUAL STIMULATION DEVICE USING LIGHT THERAPY AND VIBRATION**
VORRICHTUNG ZUR SEXUELLEN STIMULIERUNG MIT LICHTTHERAPIE UND VIBRATION
DISPOSITIF DE STIMULATION SEXUELLE UTILISANT LA PHOTOTHÉRAPIE ET LA VIBRATION

(30) Priority: 14.03.2012 US 201261610899 P
(43) Date of publication of application: 21.01.2015
(73) Proprietor: Zipper, Ralph, Melbourne, FL 32935 (US)
(72) Inventor: Zipper, Ralph, Melbourne, FL 32935 (US)
(74) Representative: Heinze, Ekkehard
(86) International application number: PCT/US2013/031688
(87) International publication number: WO 2013/138658

(56) References cited:
- WO-A2-2011/159906
- DE-U1-202009 011 528
- US-A1- 2003 199 946
- US-A1- 2003 232 303
- US-A1- 2005 197 682
- US-A1- 2006 084 837
- US-A1- 2008 139 980
- US-A1- 2009 093 673
- US-A1- 2009 099 413
- US-A1- 2010 174 136

## Description

### BACKGROUND OF THE INVENTION

### 1. Field of the Invention

The improved sexual stimulation device of the invention relates generally to the field of female sexual stimulation devices, more specifically, handheld sexual stimulation devices using vibration techniques in order to achieve stimulation.

### 2. Background Art

A variety of handheld sexual stimulating devices have been described in the art, many of which are commercially available, and some of which have been the subject of patents. The devices of the prior art may combine mechanical vibration with other stimulation, such as heat. For example, a vibratory therapy device comprising a lightweight portable housing encasing a heater/vibrator assembly for imparting vibratory action to a massaging head, together with heated airflow, is described in U.S. Pat. No. 4,722,326. The device includes a plurality of interchangeable massage heads having different surface textures or configurations.

An electric vibrating or massaging device having a plurality of detachable attachments for use in marital or sexual orgasmic therapy is also taught in U.S. Pat. No. 5,067,480. This device includes a stimulator that rotationally oscillates through a range of angles for stimulation of appropriate body areas in marital or sexual orgasmic therapy.

None of the aforementioned vibrating or massage devices combine the use of therapeutic light energy for photostimulation and or photobiomodulation and vibration in a single apparatus which provides multiple modes of use including programmable mechanical stimulation and light energy control, multiple frequencies of light energy, a plurality of vibrators or mechanical stimulators and therapeutic light sources which are intended to increase blood flow, improve tissue health, and decrease microbes and therefore improve sexual stimulation and genital health, and numerous pre-programmed modes of operation. "Mechanical stimulation", as used herein, is defined as mechanical manipulation of a body surface that is perceivable by a person and wherein said mechanical manipulation is achieved by any means including but not limited to vibration, sonic pulses, rubbing, tapping, pressure, pressure that varies in intensity, and any other form of mechanical manipulation of surface body tissue in a perceptible manner. "Therapeutic light", as used herin, refers to any light, visible or not visible, that exerts an effect to the biologic or chemical status of the tissue or microbe to which it is applied, with such effect or effects being other than that of the activation or modification photosensitive receptors of the human eye. Examples of therapeutic effects may include but not be limited to alteration is cellular respiration, alteration or activation of enzyme or enzyme pathways, alteration in mitochondrial activity, the production or reduction in adenosine triphosphate or similar molecules, the production or reduction of nitric oxide or similar chemicals, changes in blood pressure, muscle relaxation, muscle contraction, cellular activation, modification of the inflammatory response, modification of the healing response, hastening of microbe activity, alteration of microbe activity, and microbe death. Therapeutic light may be referred to as photostimulation when the effects of said light are stimulating in nature. Therapeutic light may be referred to as photomodulation or photobiomodulation when the effects of said light are other than stimulatory or beyond stimulatory. A device according to the preamble of claim 1 is known from DE 20 2009 011528 U1 and from US 2006/084837 A1.

### BRIEF SUMMARY OF THE INVENTION

According to the invention, a device as claimed in claim 1 is provided.

In the prior art female sexual stimulation devices were designed to vibrate and stimulate the nerves of sexually sensitive areas of the female anatomy, for instance the clitoris or Gräfenberg Spot (G-spot). Although effective, this method of stimulation typically bypasses or rapidly moves through one of the most important phases of the sexual response cycle which is the arousal phase. This phase, also known as the excitement phase, is characterized by increased blood flow to the clitoris and vagina as well as an increase in muscle tone to the vagina and anus. The present invention utilizes therapeutic light energy to enhance genital blood flow. The ability of specific wavelengths of light to stimulate blood flow through the application of light energy is well established throughout medical literature. The application of light energy to certain parts of the body may improve blood flow and may therefore improve sexual response with or without a partner. Improved blood flow can also extend a woman's sexual lifespan. The apparatus of the invention causes greater clitoral swelling and signs of improved genital blood flow, which may result in enhanced or more frequent orgasms (or both). Additionally, the present invention may utilize specific wavelengths of therapeutic light to decrease vaginal and vulvar bacteria and fungi populations. The medical and environmental literature is replete with data demonstrating specific wavelengths of light to be bactericidal, bacterostatic, fungicidal and fungistatic. The application of such light energy to the vagina, vulva, clitoris, and penis may reduce bacterial and fungal infections that may be caused by the use of stimulating devices or exist unrelated to such.

It is preferred that, when a preferred embodiment of the improved sexual stimulation device of the invention is used in a best mode, the vaginal finger directly applies vibration and light stimulation on or near the Gräfenberg Spot, or G-spot, of a female user, and the clitoral finger directly applies vibration and light stimulation on or near the clitoris of a user. In this manner, vibration and light stimulation are directly applied to the areas of the body known to result in maximum arousal and sexual stimulation. The improved sexual stimulation device of the invention may be used in numerous orientations and modes which are limited only by the imagination of the user or the user's partner, and are therefore not to be limited by the best mode described herein.

### BRIEF DESCRIPTION OF THE DRAWINGS

The accompanying drawings, which are incorporated into and form a part of the specification, illustrate one or more embodiments of the present invention and, together with the description, serve to explain the principles of the invention. The drawings are only for the purpose of illustrating the preferred embodiments of the invention and are not to be construed as limiting the invention as claimed. The drawings of the various figures may not be too scale and some features may be exaggerated or minimized in order to clearly describe the invention. In the drawings:
**Fig. 1** depicts a perspective view of a preferred embodiment of the improved sexual stimulation device of the invention.
**Fig. 2** depicts a side view of a preferred embodiment of the improved sexual stimulation device of the invention.
**Fig. 3** depicts a front view of a preferred embodiment of the improved sexual stimulation device of the invention.
**Fig. 4** depicts a rear view of a preferred embodiment of the improved sexual stimulation device of the invention.
**Fig. 5** depicts an exploded view of a preferred embodiment of the improved sexual stimulation device of the invention.
**Fig. 6** depicts a cross-sectional view of a preferred embodiment of the improved sexual stimulation device of the invention, further depicting the location of the electrical components of the invention.
**Fig. 7a** depicts a top view of the vaginal printed wiring board of a preferred embodiment of the invention, depicting a clitoral source group in electrical and mechanical communication with a clitoral finger printed wiring board.
**Fig. 7b** depicts a top view of a clitoral printed wiring board of a preferred embodiment of the invention, depicting a vaginal source group in electrical and mechanical communication with a vaginal finger printed wiring board.
**Fig. 8** depicts a functional block diagram of a preferred embodiment of the improved sexual stimulation device of the invention.
**Fig. 9** depicts exemplary light source patterns for an exemplary embodiment of the invention which comprises light source groups having a blue light source, a red light source, and a near infrared light source.
**Fig. 10** depicts exemplary vibrator patterns for an exemplary embodiment of the invention having a vaginal finger vibrator and a clitoral finger vibrator.
**Fig. 11** depicts a table of preferred modes of vibration and light stimulation for a preferred embodiment of the improved stimulation sexual device of the invention.

### DETAILED DESCRIPTION OF THE INVENTION

The following documentation provides a detailed description of the invention.

The invention is a device primarily intended for sexual stimulation of a female user. As used herein, "mechanical stimulation" shall mean any energy that exerts a perceivable movement to the tissue upon which it is applied.

The light source or light sources may be automatically powered when the mechanical energy element of the device is powered to an ON state, or may be configured so as to operate independently from the source or sources of mechanical energy. A plurality of light sources provide light energy that exits the device at one or more points, at a point along the length of the vaginal finger and also at a point along the length of the clitoral finger, which may irradiate the tissue in the vagina and may irradiate the body of the user on or near the clitoris or vulva. A preferred embodiment of the improved sexual stimulation device of the invention may comprise both a vaginal vibrator and a clitoral vibrator. The vaginal vibrator and clitoral vibrator may comprise any vibrating element small enough to fit within the envelope of vaginal and clitoral fingers of the invention. In a preferred embodiment, the vaginal vibrator and clitoral vibrator may be defined as a DC motor with offset weights mounted on the motor shaft such that the center of mass of the weight is offset from the axis of rotation of the motor. When such motors are powered to an on state, a vibration results from the offset nature of the center of mass of the weight mounted onto the vibrator motor shaft. While the vibrators of the preferred embodiment may exhibit any rate of vibration, a preferred range of vibration rate is 5,000 to 25,000 rpm. Such offset vibration motors are well known in the art for use as sources of vibration. One such vibration motor is supplied by Shenzhen Kinmore Motor Co. of Guangdong, China, part number FF-N20VA-09170 R6x4.8. However, any small source of vibration or mechanical energy may be used as the vibration elements of the invention. For instance, sonic pulses have been shown to have a vibratory effect, and may be used as a vibration source in either the vaginal finger, the clitoral finger, or both. An alternate embodiment of the invention may thus use at least one sonic pulse generator as a source of vibration; or, alternatively, may comprise vibration means that produce other forms of mechanical stimulation such as, for example, rubbing, tapping, pressure, and pressure that varies with time.

The invention may emit therapeutic light energy that irradiates the areas on or near the Gräfenberg Spot inside the vagina, and may also irradiate the area on or near the clitoris and or elsewhere on the vulva or perineum.. The Gräfenberg Spot, often called the G-Spot, is defined as a bean-shaped area of the vagina. Some women report that it is an erogenous zone which, when stimulated, can lead to strong sexual arousal, powerful orgasms and female ejaculation. The G-Spot is typically described as being located one to three inches (2.5 to 7.6 cm) along the front (anterior) vaginal wall between the vaginal opening and the urethra and is a sensitive area. It is one objective of the invention to irradiate the G-spot and the area in proximity to the G-spot with light energy. However, the emitted light energy may also be directed through a broader or de-focused beam to surrounding tissues of the vagina. The concentration or de-focusing of light energy on specific areas such as the Gräfenberg Spot or clitoris may be achieved by varying the placement of the light energy exit points of the invention, which may be a single or a plurality of exit points, and which may be covered by a wavelength transparent material, at appropriate positions on the apparatus in order for the exiting light energy to impact the intended area or areas. Similar alterations in light projection may be created by placing a lens or lenses over the light source or sources.

The light energy delivered by the device may be within a plurality of different ranges of wavelength. The light sources of the invention may be any light source such as Light Emitting Diode (LED), laser diode, organic LED (OLED) or any other light source which is compact enough to be enclosed in the apparatus. The wavelength of the light source, or sources, of the invention may be any wavelength, but is preferably in the range from 400nm to 1000nm. A plurality of light sources, which may be of different wavelengths, is utilized; it is not necessary that each of a plurality of sources emit light of the same wavelength. A preferred embodiment utilizes a plurality of light sources that, taken together, enables the invention to emit more than one wavelength range of light energy. The light energy used may be such to limit therapeutic penetration to less than 5mm from the tissue surface and to have greater than 50% drop off of energy beyond such depth because the intended nerves and vessels that are the subject of stimulation are essentially superficial. Light energy may be delivered in continuous or pulsed form, where the pulses may take any shape and be of any duration. The power level of the light energy emitted by the light sources of the invention may be modulated in any fashion such as, for example, pulse width modulation, but preferably is controlled by increasing or decreasing the electrical current through the light source. "Light source" as used herein means a device that converts electrical energy to light energy such as an LED, OLED semiconductor laser, or any other device that exhibits this characteristic.

More specifically, a preferred embodiment of the improved sexual stimulation device of the invention comprises more specific ranges of bandwidth and output power of the light sources which are now described. The selection of bandwidth and output power of the therapeutic light sources for this preferred embodiment is based upon the demonstrated effects of photostimulation or photobiomodulation. It has been demonstrated that light sources emitting energy in the infrared and near infrared spectrum may provide photostimulation and photobiomodulation for the temporary relief of minor muscle and joint pain, muscle spasm, pain and stiffness, by promoting relaxation of the muscle tissue and temporarily increasing local blood circulation. As used herein, the term "light therapy" refers to the use of one or more light sources of any type that emits light with a wavelength between about 400 and 1000nm.

Light therapy induces a variety of photo-thermal and photo-chemical processes in the body. Infrared light, near infrared light, and red light affect cellular mitochondria and activate surrounding enzymes resulting in the release of Nitric Oxide , ATP, and trigger photo neurological responses which result in changes in local pressure, temperature and permeability of cellular membranes, and stimulation of the immune, lymphatic and vascular systems. Organic nitrates are used every day in emergency rooms around the world to improve blood flow to the heart. There is also an inverse relationship between nitric oxide pathways and atherosclerosis. Patients with impaired NO pathways seem to have higher amounts of blood vessel plaques (narrowed blood vessels with poor blood flow and oxygen delivery to tissues). Over time, improved NO pathways may decrease atherosclerosis and create healthier "younger" blood vessels. Within the 400 to 1000nm range is therapeutic blue light. Blue light energy has a bactericidal and bacteriostatic effect and has been shown to kill and disrupt growth of pathogenic bacteria. This inactivation mechanism, known to be oxygen dependent, is thought to be a result of the photoexcitation of naturally occurring endogenous porphyrins, which act as endogenous photosensitizers within the bacterial cells. This porphyrin excitation leads to energy transfer and, ultimately, the production of highly cytotoxic, oxygen-derived species, most notably, singlet oxygen. Although ultraviolet light is also lethal to many pathogenic bacteria, as we know, UV light is very detrimental to the skin. Blue light is much safer. Over time, blue light can safely lead to a reduction in pathogenic bacteria on skin and mucous membrane surfaces. Near IR light, also within this preferred spectrum of therapeutic light, damages the genetic material inside of fungus (DNA). DNA damage leads to impaired fungal growth. Over time the yeast population on skin and mucous membranes decreases. The use of IR light to treat toe nail fungus is now common medical practice.

Wavelength and power density are the two most prominent factors that determine the effectiveness of a light therapy source. The wavelength of the light source determines the absorption rate and penetration depth of the light energy in biological tissue. The power density of the light source, in combination with its wavelength, determines the effect the emitted light produces on body tissue, bacteria, and fungi. The effects of light therapy with therapeutic light have been studied for applications in, for example, pain relief and tissue healing: in, fact, about 2,700 clinical reports have been published in peer reviewed technical journals or conference proceedings with more than 70% of the reports indicating that light therapy is effective for tissue healing and pain relief. Clinical reports demonstrate that light therapy is effective, for instance, for the relief of muscle and joint pain, muscle spasm, pain and stiffness associated with arthritis, by promoting relaxation of the muscle tissue and temporarily increasing local blood circulation, without side effects. An even greater amount of data exist for the effects of light on bacteria and fungi.

It has also been shown that various pulse formats have a positive effect on photostimulation. Off-times between light pulses of 50ms to 500ms may have the greatest effect on cell organelles and plasma membranes. It is postulated that when this range of pulse formats is used, the re-oxidation of cytochrome c oxidase is optimized. This optimization leads to increase energy in the cell leading to improved blood flow and tissue repair. Pulsing of the light source is used to optimize the photobiological response though a temporal optimization as well as dosage control. Optimal dosages of light range from of 0.001 J/cm² up to 3000 J/cm². These dosages can be controlled through changing the intensity of the light source, changing the irradiation time, and by light source pulse shape and timing. Therapeutic results can be seen using intensities ranging from 0.001 W/cm² to 100 W/cm².

Studies have concluded that photostimulation can increase blood flow. Increased blood flow is critical for bodily functions and is an important factor in the excitement phase of sexual stimulation. Photostimulation enhances vasodilatation and proliferation of the microvasculature as well as increases the level of oxygen content to tissue of the irradiated area, in this case, the G-spot area, and the area surrounding the clitoris. In a recent study which compared a photostimulated group of human test subjects to a non-photostimulated group of human test subjects, the photostimulated group increased blood flow to the exposed area for over 12 hours. This study showed the capillaries of the human subjects were enlarged and thus blood flow increased as a result of photostimulation. Photostimulation activates the powerhouse for the cell to increase the rate at which it produces energy. This activation increases the production of critical biochemical substances such Nitric Oxide (NO) and Vascular Endothelial Growth Factors (VEGF). These growth factors also promote new blood vessel growth. These biological responses to light therapy explain why photostimulation can have both an immediate and cumulative effect on a subject's level of sensation during sexual stimulation. As noted above, it has also been shown in laboratory studies that light energy in certain bandwidths may also have an anti-bacterial effect. Clinical studies have verified this antibacterial effect on patients for light sources between 405nm and 420nm. It has also been shown that a combination of blue and red output from LEDs can be a very effective treatment of both inflammation and antibacterial results. Recent studies have shown that blue LEDs irradiating the skin may result in a seven to fifteen times increase in the NO level in tissue as deep as 18mm below the skin's surface.

Based on the effects of light therapy on human tissue, it is a feature of a preferred embodiment of the invention that blue light sources emitting light energy in the ranges of 400nm to 515nm or 530nm to 670nm at a output of at least 300 millicandelas peak with a half-power output angle of +/- 60 degrees, red light energy in the ranges of 610nm to 640nm at a output of 300 millicandelas peak with a half-power output angle of +/- 60 degrees, and infrared light energy in the range of 820nm to 880nm at an output radiant flux of at least 300mW peak with a half-power output angle of +/- 60 degrees with each light source irradiating in pulses of equal on and off times between 50msec and 500msec, is one embodiment of many that creates the desired effects on tissues associated with the use of adult pleasure objects. Said effects are including, but not limited to, improved blood flow, improved blood vessel health, tissue regeneration, tissue tightening, improved cellular respiration, improved lubrication, decreased bacteria, decreased fungi, enhanced arousal and even pain relief. These effects may be directly caused in a user by use of the improved sexual stimulation device of the invention, as described herein. It is to be noted that this is just one of many embodiments of the invention, and any number of light sources emitting light energy within the range of 400nm to 1000nm at any radiant flux or millicandela output and angle may be used in any light source group of the invention. In order to emit the various bandwidths of light energy, each light source group may comprise more than one type of light source. For example, in the preferred embodiment described for which multiple frequencies of light are emitted by a light source group, a light source group may comprise light emitters which are defined as a plurality of LEDs, a plurality of lasers, or a plurality of light sources comprising a combination of LEDs and lasers. The light sources of the invention may be adapted to provide continuous output or provide output energy in pulses of any wave shape. "Half power angle" as used herein means the off-axis angle where the light source's luminous intensity is half the intensity at direct on-axis view. The axis of the light source is defined as the line of the vector of maximum intensity emanating from the light source.

In the various embodiments, the light energy may be pulsed on and off and may be any output energy with the ranges of output energies set forth herein. These light source output wavelengths may be administered in pulses that are in phase or out of phase as between light sources, and may be of any timing desired. It is an aspect of the invention that such light source pulse shape and timing may be, for some embodiments, programmable by use of a controller in communication with the light sources. The controller may be programmed by the user to produce a desired pulse shape and timing for the light sources of the invention. Alternatively the controller may be preprogrammed by a non-user.

In another preferred embodiment, these wavelengths, pulse widths, rest periods, and output power may be combined with mechanical energy such as that provided by an unbalanced vibration motor typical to that found in an adult sex toy, or any other method of mechanical energy delivery known in the art. In this manner, light therapy and mechanical stimulation, and, more specifically, vibration, may be applied together, or in alternating patterns of the user's choice, in order to achieve a desired stimulation effect.

In a preferred embodiment of the device, the light sources may be delivered to the tissue through an optical diffusing element, such as, for example, a silicone cover, to assure a homogeneous exposure of light to the selected tissue. In an alternate embodiment the device, the light sources may produce a selected pattern of optical energy to deliver optical energy to specific spots to provide differential exposure to unique spots on the tissue.

Yet further alternate embodiments will have preprogrammed light energy pulse patterns and or vibration patterns. The preferred embodiment comprises separate buttons for activating and choosing vibrations patterns and or light energy patterns.

In some of the specific preferred embodiments described herein, the mechanical stimulation example given is described as mechanical vibration produced by, for example and not by way of limitation, an offset vibrator motor. However, it is to be understood that the scope of the alternate embodiments of the invention includes all types of mechanical simulation devices and methods known in the art and which are adaptable to the invention without undue experimentation, such as sonic vibrators and mechanical manipulators of any type that produce rubbing, tapping, pressure, pressure that varies in intensity over time, and all other methods for mechanical stimulation.

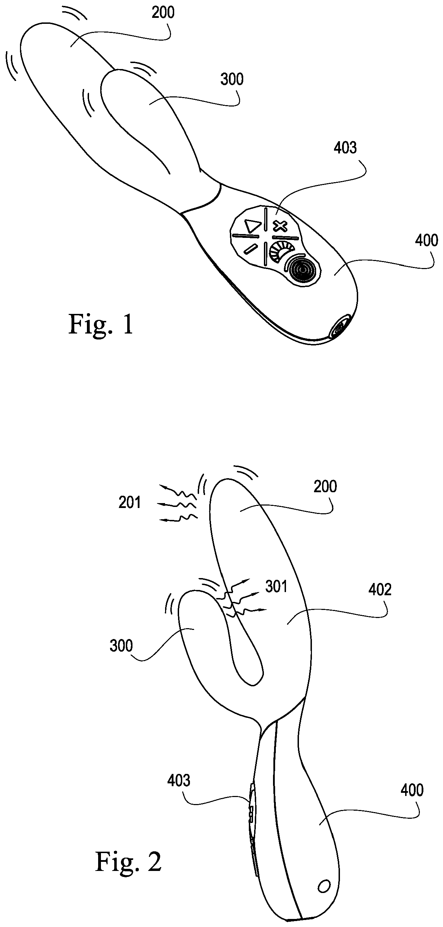

Referring now to **Fig. 1****,** a preferred embodiment of the sexual stimulation invention is shown in perspective view. The invention comprises vaginal finger **200,** clitoral finger **300,** handle **400** and keypad **403.** The device is intended to be used primarily by a female user, who, in the most general case, would hold the invention with one hand or both hands by handle **400** and slidingly engage vaginal finger **200** in a vagina such that vaginal finger **200** applies vibration and vaginal light energy **201** within the vagina at or near the G-spot, while clitoral finger **300** may apply clitoral light energy **301** and vibration at or near a clitoris. The application of light energy and vibration at or near the G spot and clitoris in continuous fashion, or in patterns of vibration and light therapy as may be programmed into the invention, operate to cause more rapid and intense stimulation than is possible without the invention. One exemplary method may comprise the following steps: applying lubrication, a lubricating light coupling agent or a combination of such agents as desired to vaginal finger **200** or clitoral finger **300,** or both, slidingly engaging vaginal finger **200** in a vagina while resting clitoral finger **300** on or near a clitoris, turning the vaginal light source group **202** and clitoral light source group **302** of the apparatus ON, turning the vaginal vibrator **204** and clitoral vibrator **304** of the apparatus ON, and holding and applying movement as desired by grasping handle **400.** Alternatively, light source groups and or vibrators may be turned on prior to contact of fingers to body tissue. The invention may further comprise control circuitry located in the handle **400** of the invention which may be used to operate the invention in one or more of many possible operational modes which are discussed further herein. Keypad **403** may cover the internal circuitry of the invention in such a manner as to provide environmental protection for the circuitry while at the same time allowing a user to control the operation of the invention by pressing on keypad **403** which is in physical contact with and engages switches **500** in electrical communication with the control circuitry. In normal use, handle **400** remains outside the body of the user. It can further be seen from **Fig. 1** that vaginal finger **200,** clitoral finger **300** and handle 400 form a unitary structure. In an alternate embodiment, clitoral finger **300** may not be present: in this alternate embodiment vaginal finger **200** and handle **400** form a unitary structure.

Referring to **Fig. 2****,** vaginal finger **200** and the clitoral finger **300** are shown as vibrating, as during normal use in the preferred embodiment shown. Vaginal light energy **201** may irradiate body tissue at or near the G-pot as it radiates from a side of distal end of vaginal finger **200** through flexible cover **402.** Clitoral light energy **301** may irradiate body tissue at or near the clitoris as it radiates from the distal end of clitoral finger **300** through flexible cover **402.** Flexible cover **402** may cover vaginal finger **200** and clitoral finger **300** and may, in a preferred embodiment, provide a hypo allergenic cleanable covering for vaginal finger **200** and clitoral finger **300.** Flexible cover **402** may be fabricated from any material suitable for this purpose and transmissive at frequencies of 400nm to 1000nm including, but not limited to, silicone. The use of silicone in this preferred embodiment exhibits reduced friction characteristics which facilitates sliding engagement of vaginal finger **200** into the vagina of a user and also facilitates reduced frictional properties when clitoral finger **300** is in contact with the body of a user on or near the clitoris. Keypad **403** covers switches **500** (not shown in **Fig. 2**) which, in this preferred embodiment, are located within handle **400.** The anterior side of keypad **403** is directly pressed by a user, for example by a user's finger, in order to activate controls of the invention. The posterior side of keypad **403** comprises keyboard button nipples which engage switches **500** (not shown in **Fig. 2**) disposed on a surface of controller printed wiring board **418** (not shown in **Fig. 2**) when a user presses any of the depressible control buttons disposed on the anterior side of keypad **403.** In this way, a user may control the improved sexual stimulation device **100** to power the device on or off, and to command to operate in any of the modes described herein.

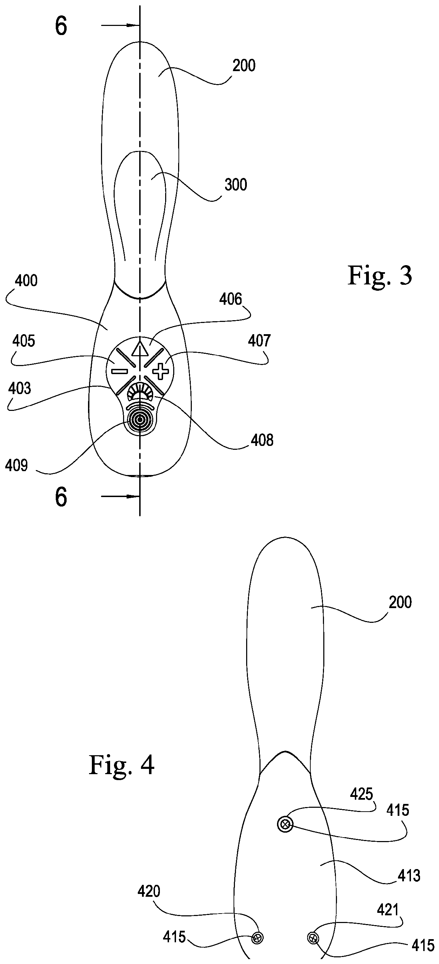

Referring now to **Fig. 3****,** a front view of a preferred embodiment of the improved sexual stimulation device **100** of the invention is shown. Keypad **403** comprises a first depressible control button **405,** a second depressible control button **406,** a third depressible control button **407,** fourth depressible control button **408,** and fifth depressible control button **409** which are operated by a user to control the device. As described further herein, pressing downward on a depressible control button of the invention causes a keypad button nipple **428** (not shown in **Fig. 3** but described further herein) disposed on the underside of keypad **403** to engage switches **500** (not shown in **Fig. 3**), which are in electrical communication with controller **501,** providing commands as desired by the user to controller **501** (not shown in **Fig. 3**). The functions of the five depressible control buttons depicted in **Fig. 3** are discussed hereinbelow. It is to be noted that the operational modes described herein are exemplary. Due to the programmable nature of controller **501** the depressible control buttons of the invention may be programmed to provide any combination of vibrator or light source on state, vibrator or light source off state, intensity of light, intensity of vibration, selection of pre-programmed vibration pulse shapes or continuous operation, selection of pre-programmed light energy pulse shapes or continuous operation, test modes, light energy and vibration display modes, and the like, may be programmed into the invention, selected and operated by a user pressing the depressible control buttons of the invention, which may be programmed to command any of these operations. Mechanical stimulating components other than vibrating motors may be substituted for vibrating motors and similarly controlled.

Referring now to **Fig. 4****,** a rear view of a preferred embodiment of the improved sexual stimulation device **100** of the invention is depicted. Shown in **Fig. 4** are first cavity **420,** second cavity **421** and third cavity **425,** which may provide recessed insertion points for handle rear cover fastener **415** which are inserted through first cavity **420,** second cavity **421** and third cavity **425** and are threadingly engaged into receiving female threads located on main support structure **412** (not shown in **Fig. 4**), and are therefore used to hold handle rear cover **413** into place. Alternatively, such cavities may be replaced by molded pins communicating with glue bosses rather than screw bosses.

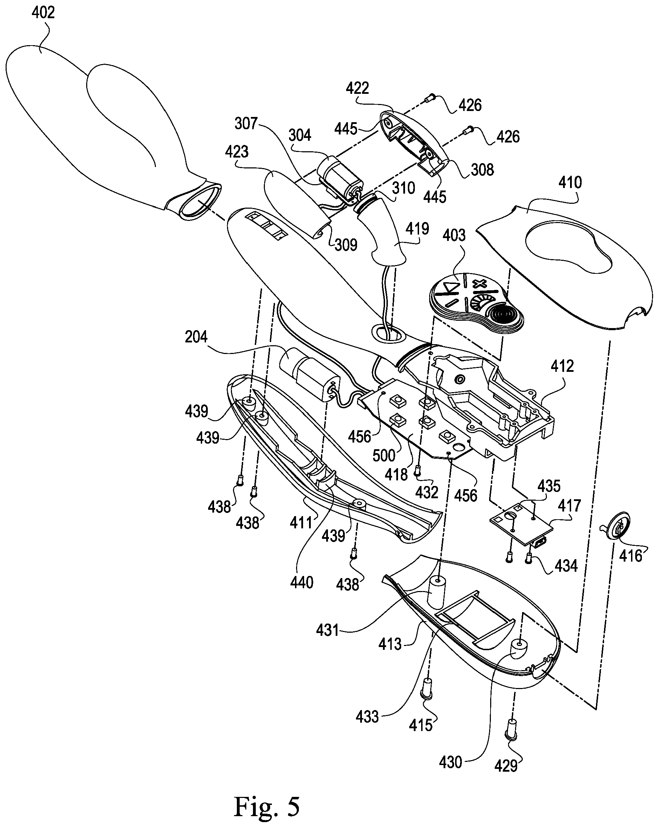

Referring now to **Fig. 5****,** an exploded view of the preferred embodiment of the improved sexual stimulation device **100** of the invention is depicted. It is to be noted that, while a specific structure is shown in **Fig. 5****,** there are many structures which may be covered by the claims.

Still referring to **Fig. 5****,** main support structure **412** is an elongate mechanical structure, preferably molded from plastic, but also, if desired, cast from metal or machined from any stable substantially rigid material such as plastic, phenolic, or other materials known in the art. Main support structure **412** provides support and attachment points for the various components and internal elements of the invention. The attachment points may generally be female bosses which are adapted to receive male threaded fasteners, and which may be threaded with female threads or may receive self-threading male threaded fasteners. All such female bosses may also serve as adhesive bosses to receive male pins and adhesive. Bosses, screw and pins may be decreased in number or removed in entirety if ultrasonic or similar welding is used to secure surfaces.

Still referring to **Fig. 5****,** the attachments of keypad **403** and handle front cover plate **410** are discussed. Keypad **403** rests upon main support structure **412** and may be preferably bonded into place thereon using any suitable adhesive for bonding keypad **403,** which may be fabricated from, for example, silicone, silicone compounds or any flexible polymer material. Alternatively, keypad **403** may be allowed to simply rest in place, sandwiched between handle front cover plate and main support structure **412.** Keypad **403** may comprise a plurality of keypad button nipples **428** (not shown in **Fig. 5**) that act to engage switches **500** when a depressible control button is depressed. Handle front cover plate **410** may be attached to main support structure **412** by handle front cover fastener **429,** which may reside in handle front cover fastener recess **430,** with the male threaded portion of handle front cover fastener **429** protruding thru handle front cover fastener recess **430** and being threadingly received by a female boss disposed on the underside of handle front cover **410** (not shown in **Fig. 5**). Likewise, handle front cover plate **410** is attached at its forward end by second handle front cover fastener **432** which may pass through an opening in main support structure **412** to be threadingly received by a female boss disposed on the underside of handle front cover **410** (not shown in **Fig. 5**). In this manner, keypad **403** and handle front cover plate **410** are held in place, allowing keypad button nipples **428** (not shown in **Fig. 5**) to rest, or nearly rest, upon switches **500** such that depression of any of the depressible control buttons **405 - 409** cause activation of a switch underneath, sending commands to controller **501** (not shown in **Fig. 5****)** on controller printed wiring board **418.**

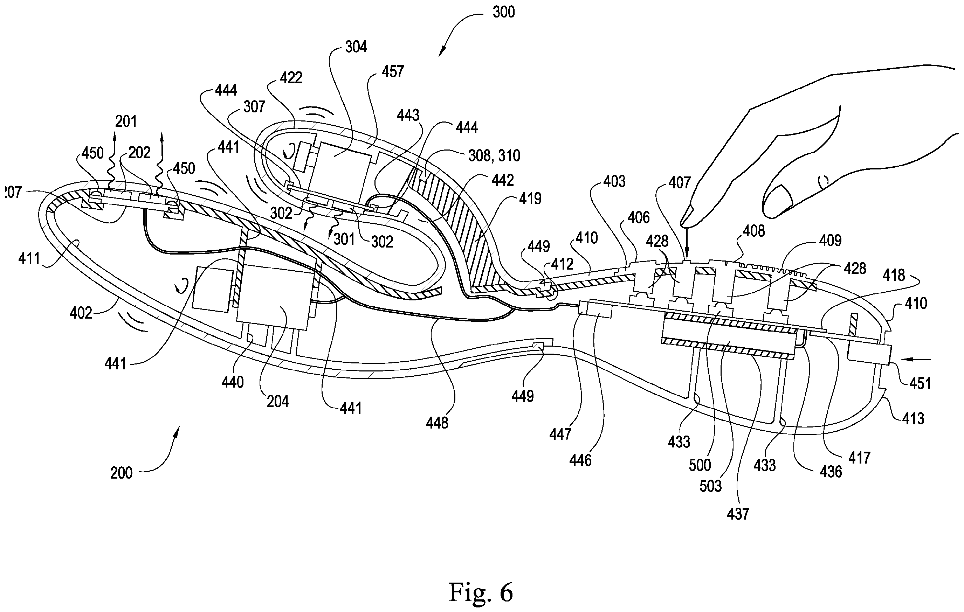

Referring still to **Fig. 5****,** the attachment of controller printed wiring board **418,** USB port printed wiring board **417,** handle rear cover **413,** and battery **503** (not shown in **Fig. 5**) is discussed. USB port printed wiring board **417** may be structurally attached to main support structure **412** by USB printed wiring board fasteners **434,** which pass through clearance holes in USB port printed wiring board **417** to be threadingly received by female bosses disposed on the underside of main support structure **412** (not shown in **Fig. 5**). USB printed wiring board fasteners **434** may be electrically connected to controller printed wiring board **418** by wires or direct solder connection between them at USB printed wiring board solder attachments **435** or equivalent electrical connection structures known in the art. Alternatively, USB port printed wiring board **417** may be fabricated as a unitary element of controller printed wiring board **418.** USB port printed wiring board **417** may comprise an electrical Universal Serial Bus (USB) charging port connector **451** to a charging or programing mating connector; however, the form of the electrical connection may take any form known or conceived in the electrical arts such as USB, micro-USB, custom design connection, or any other standard electrical connection suitable to fit within the envelope allowable. Controller printed wiring board **418** attaches to main support structure **412** by threaded fasteners (not shown in **Fig. 5**) passing through clearance holes **456** to be threadingly received by female bosses disposed on the underside of main support structure **412** (not shown in **Fig. 5**). Rear handle cover **413** is attached to main support structure **412** by handle rear cover fastener **415,** which resides in handle rear cover fastener recess **431** with the male threaded portion of handle rear cover fastener **415** protruding through handle rear cover fastener recess **431** and being threadingly received by a female boss disposed on the underside of main support structure **412** (not shown in **Fig. 5**). Battery **503** (not shown in **Fig. 5** for clarity sake, but shown in **Fig. 6**) may be sandwiched in place between the underside of controller printed wiring board **418** and battery support structure **433,** and may be electrically connected with battery wires **436** (not shown in **Fig. 5****,** but shown in **Fig. 6**) to controller printed wiring board **418** by a solder or other standard technique for making electrically conductive connection. Battery **503** may further comprise a battery compressive covering **437** such as compressible foam (not shown in **Fig. 5** but shown in **Fig. 6**), or layers of compressive material fabricated from any suitable compressive material, so that it is sandwiched and held in place between the underside of controller printed wiring board **418** and battery support structure **433** with a compressive fit to prevent movement of the battery during shipping and use. Charging port connector **451** may be wired to communicate directly with controller **501** to facilitate programming. Likewise, a Bluetooth® chip and or WiFi chip may be used for similar purpose. Alternative methods of charging well known in the art such as inductive charging may be substituted for USB charging. The invention may further comprise an inductive charging circuit as is known in the art, such that the device may be charged by simply placing it in an inductive charging cradle.

Still referring to **Fig. 5****,** vaginal finger rear cover plate **411** may attach to main support structure **412** by vaginal finger rear cover fasteners **438** which reside in vaginal finger rear cover recesses **439** with the male threaded portion of handle rear cover fastener **438** protruding through vaginal finger rear cover recesses **439** and being threadingly received by female bosses disposed on the underside of main support structure **412** (not shown in **Fig. 5**). Vaginal vibrator **204** may be supported on its underneath side by vaginal finger vibrator retaining structure **440** when vaginal finger rear cover plate **411** is attached in place. Vaginal vibrator **204** is further held in place by main support structure vaginal supports **441** (not shown in **Fig. 5** but shown in **Fig. 6**). Vaginal vibrator **204** may be engaged with main support structure vaginal vibrator supports **441** with a press fit engagement and for further retention may be bonded into place with adhesives.

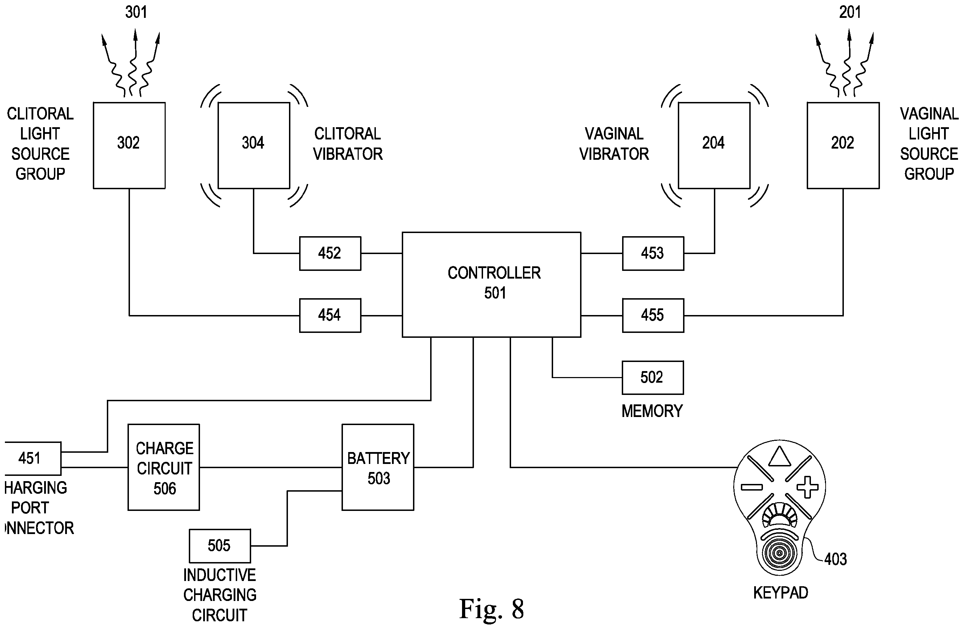

Still referring to **Fig. 5****,** clitoral finger base **419** rests upon main support structure **412** and is held in place by operation of flexible cover **402** which may provide, preferably, a covering with a slight compressive fit over clitoral finger **300** as well as vaginal finger **200.** Clitoral finger base **419** is preferably fabricated from a flexible material such as, for instance, silicone or any similar flexible material. A preferred embodiment of the invention further comprises a clitoral finger first cover **422** and a clitoral finger second cover **423** of similar opposed cross section such that, when brought together as shown in **Fig. 5** create a substantially smooth shape. While a preferred shape for the assembled clitoral finger is depicted in **Fig. 8****,** the shape of clitoral finger **300** may be any shape suitable for pleasant contact on or near the clitoris of the user. Clitoral vibrator **304** may be attached to clitoral finger printed wiring board **307** (not shown in **Fig. 5**), upon which clitoral light source group **302** (not called out in **Fig. 5**) may also be mounted with electrical connection thereto. Clitoral finger printed wiring board **307** is held in place in clitoral finger vibrator structure **457** by clitoral finger printed wiring board retaining structure **444** (not shown in **Fig. 5****,** but shown in **Fig. 6**) formed in clitoral finger first cover **422** which substantially forms a groove adapted to accept clitoral printed wiring board **307** in a press fit engagement. Clitoral vibrator **304** may be attached to clitoral finger printed wiring board **307** by adhesive bonding or any other means of attachment known in the mechanical arts. Clitoral finger first cover **422** is attached to clitoral finger second cover **423** by clitoral finger fasteners **426** which reside in clitoral finger first cover fastener recesses **445** with the male threaded portion of clitoral finger fasteners **426** protruding through clitoral finger first cover fastener recesses **445** and being threadingly received by female bosses disposed on an inside surface of clitoral finger second cover **423** (not shown in **Fig. 5**). Clitoral finger base **419** may further comprise clitoral finger base retaining groove **310** which is adapted to receive clitoral finger first cover retaining ring half **308** and clitoral finger second cover retaining ring half **309** such that, when clitoral finger fasteners **426** are installed and tightened, clitoral finger first cover retaining ring half **308** and clitoral finger second cover retaining ring half **309** are brought together to lock the assembled clitoral finger **300** components into place by operation of the retention properties of clitoral finger base retaining groove **310** holding clitoral finger first cover retaining ring half **308 and** clitoral finger second cover retaining ring half **309** in place, as is shown in cross section view in **Fig. 6****.**

Still referring to **Fig. 5****,** main support structure **412,** handle front cover plate **410,** handle rear cover **413,** vaginal finger rear cover plate **411,** clitoral finger first cover **422,** and clitoral finger second cover **423** may be fabricated from any material rigid enough to structurally support the assembly and repeated use of the improved sexual stimulation device **100** of the invention. Preferably, these components may be fabricated from any plastic material in order to facilitate ease of manufacture and minimize weight. Still within the scope of the invention, but less preferred, are other rigid materials such as metals, phenolic materials, organic materials, or any other materials rigid enough to support the assembly and repeated use of the invention. Such materials are known in the art.

Referring now to **Fig. 6****,** a cross sectional view of a preferred embodiment of the improved sexual stimulation device **100** of the invention is depicted. Keypad **403** is shown as being pressed by the finger of a user by depressing, for example, third depressible control button **407** which causes a keypad button nipple **428** to depress and activate the corresponding switch **500,** which is mounted on controller printed wiring board **418.** Thus a user may command and operate the improved sexual stimulation device **100** of the invention as desired by turning it on or off, manually changing modes, and commanding pre-programmed modes of operation, or the like. Battery **503** may be sandwiched in place between the underside of controller printed wiring board **418** and battery support structure **433,** and may be electrically connected with battery wires **436** to controller printed wiring board **418** by a solder or other standard technique for making electrically conductive connection. Battery **503** may further comprise a battery compressive covering **437,** or layers of compressive material fabricated from any suitable compressive material, so that it is sandwiched and held in place between the underside of controller printed wiring board **418** and battery support structure **433** with a compressive fit to prevent movement of the battery during shipping and use. Controller printed wiring board connector **446** mates mechanically and electrically with wiring connector **447,** enabling the connection and disconnection of clitoral finger wires **443** and vaginal finger wires **448** from controller printed wiring board **418** to facilitate assembly and repair.

Still referring to **Fig. 6****,** flexible cover **402** covers vaginal finger **200** and clitoral finger **300** with a slight compressive fit, and may be further captured in place by flexible cover retaining step **449** which may be received by a matching groove in main support structure **412** and rear handle cover **413** as shown in **Fig. 6****.** Further, Vaginal vibrator **204** may be supported on its underneath side by vaginal finger vibrator retaining structure **440** when vaginal finger rear cover plate **411** is attached in place to vaginal vibrator **204** is further held in place by main support structure vaginal supports **441.** Vaginal vibrator **204** may be engaged with main support structure vaginal vibrator supports **441** with a press fit engagement and for further retention may be bonded into place with adhesives. Vaginal finger printed wiring board **207** is held in place by vaginal printed wiring board fasteners **450** which are received by holes in main support structure **412** and which are adapted to receive vaginal printed wiring board fasteners **450,** which may be standard threaded fasteners, self-tapping or any other fastener type known in the art. When vaginal printed wiring board **207** is mounted as shown, vaginal light energy **201** from vaginal light energy source group **202** may exit vaginal finger **200** by passing through flexible cover **402** which is transmissive at light frequencies stated herein. Vaginal finger wires **448** establish electrical connection between vaginal printed wiring board **207,** vaginal vibrator **204,** and controller printed wiring board **418.** Clitoral finger printed wiring board **307** is held in place in clitoral finger **300** by clitoral finger printed wiring board retaining structure **444** formed in clitoral finger first cover **422** which substantially forms a groove adapted to accept clitoral printed wiring board **307** in a press fit engagement. Clitoral vibrator **304** may be attached to clitoral printed wiring board **307** by adhesive bonding or any other means of attachment known in the mechanical arts. When clitoral printed wiring board **307** is mounted as shown, clitoral light energy **301** from clitoral light energy source group **302** may exit clitoral finger **300** by passing through flexible cover **402** which is transmissive at light frequencies stated herein. Clitoral finger wires **443** establish electrical connection between clitoral printed wiring board **307,** clitoral vibrator **304,** and controller printed wiring board **418.** Clitoral finger base **419** rests upon main support structure **412.** Clitoral finger wires **443** pass through clitoral finger base channel **442.** Clitoral finger base **419** may further comprise clitoral finger base retaining groove **310** which is adapted to receive clitoral finger first cover retaining ring half **308** and clitoral finger second cover retaining ring half **309** such that, when clitoral finger fasteners **426** (not shown in **Fig. 6**) are installed and tightened, clitoral finger first cover retaining ring half **308** and clitoral finger second cover retaining ring half **309** (not shown in **Fig. 6**) are brought together to lock the assembled clitoral finger **300** components into place by operation of the retention properties of clitoral finger base retaining groove **310** holding the retainer ring halves, **308 and 309,** in place.

Charging port connector **451** is electrically and mechanically attached to USB port printed wiring board **417,** and protrudes through an opening formed in handle front cover plate **410** and handle rear cover **413** when they are assembled as described herein.

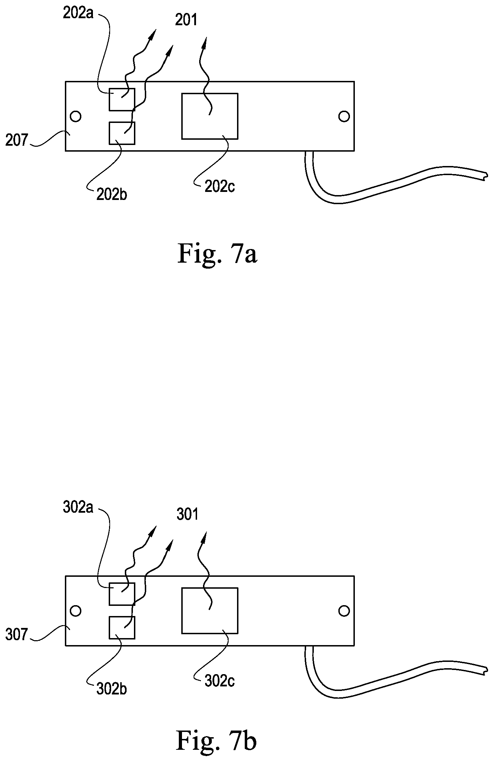

Referring now to **Fig. 7a** and **Fig. 7b****,** details of a preferred embodiment of vaginal finger printed wiring board **207** and clitoral finger printed wiring board **307** are depicted. Both of these printed wiring boards may contain one or more light sources in electrical connection with wiring that is in electrical connection with controller printed wiring board **418** as follows. Referring first to **Fig. 7a****,** vaginal finger printed wiring board **207** may comprise vaginal light source group **202** which is itself comprised of at least one light source, but preferably is comprised of a variety of light sources in order to combined a variety of frequencies of light that may be radiated as vaginal light energy **201.** Vaginal light source group **202** may comprise any number of light sources. In the preferred embodiment shown in **Fig. 7a****,** three vaginal light sources **202a, 202b** and **202c** are depicted as comprising vaginal light source group **202.** Each light source may consist of one or more diodes. Vaginal light sources **202a, 202b** and **202c** may be soldered to vaginal finger printed wiring board **207** to provide electrical and mechanical connected thereto, or may be attached to vaginal finger printed wiring board **207** in any manner known in the electrical assembly arts. In an alternate embodiment, the light sources of vaginal light source group **202** may be individually attached to main support structure **412** or its equivalent and connected with discrete wires (not shown in **Fig. 7a**) to controller printed wiring board **418.** Referring now to **Fig. 7b****,** clitoral finger printed wiring board **307** may likewise comprise clitoral light source group **302** which is itself comprised of at least one light source, but preferably is comprised of a variety of light sources in order that a variety of frequencies of light may be radiated as clitoral light energy **301.** Clitoral light source group **302** may comprise any number of light sources. In the preferred embodiment shown in **Fig. 7b****,** three clitoral light sources **302a, 302b** and **302c** are depicted as comprising clitoral light source group **302.** Each light source may consist of one or more diode. Clitoral light sources **302a, 302b** and **302c** may be soldered to clitoral finger printed wiring board **307** to provide electrical and mechanical connected thereto, or may be attached to clitoral finger printed wiring board **307** in any manner known in the electrical assembly arts. In an alternate embodiment, the light sources of clitoral light source group **302** may be individually attached to main support structure or its equivalent and connected with discrete wires (not shown in **Fig. 7b**) to controller printed wiring board **418.** In a preferred embodiment, the light sources of vaginal light source group **202** may be defined as light source **202a** and **202b,** each having a "blue" LED emitting wavelengths between 400nm to 515nm and a "red" LED emitting wavelengths between 610nm to 640nm for a total of 4 LEDs; and light source **202c** comprising an infrared LED emitting wavelengths between 820nm to 880nm. Likewise, in preferred embodiment, the light sources of clitoral light source group **302** may be defined as light as light source **302a** and **302b,** each having a "blue" LED emitting wavelengths between 400nm to 515nm and a "red" LED emitting wavelengths between 610nm to 640nm for a total of 4 LEDs and light source **302c** comprising an infrared LED emitting wavelengths between 820nm to 880nm. While these exemplary light source groups are discussed herein as regards the modes of operation of the invention, it is to be understood that the exact number of light sources in each light source group is variable, as well as are the frequencies of light emitted, the output power of the emitted light energy, and the modes of operation. Thus, a light source group may consist of two "blue" LEDs, three infrared LEDs, and LEDs with differing wavelengths and output powers other than the blue, red and infrared light sources described above, and so on. All combinations of light source types are considered within the scope of the claimed invention. Furthermore, alternate embodiments of the invention may comprise a light source or light sources disposed only in clitoral finger **300** and not in vaginal finger **200,** and vice versa. Additionally, alternate embodiments may utilize light sources known in the art that are disposed in the handle of the device or external to the device whereby therapeutic light energy is transmitted to vaginal and or clitoral finger through light tubes or fibers.

Referring now to **Fig. 8****,** an electrical block diagram of the sexual stimulation device **100** of the invention is depicted. The invention is an electro-mechanical apparatus that may comprise a vaginal light source group **202,** a clitoral light source group **302,** a vaginal vibrator **204,** a clitoral vibrator **304,** a controller **501,** a memory **502,** a charging circuit **506,** an inductive charging circuit **505,** a charging and programming port connector, such as a USB connector, **451,** and a keypad **403.** Controller **501** may reside on controller printed wiring board **418** (not shown in **Fig. 8**) and be soldered thereto. Controller **501** may be a single electrical device or a plurality of electrical devices that allow control inputs to be accepted from a user through the use of keyboard **403,** by a user depressing one or more depressible control buttons as hereinbefore described. Controller **501** may comprise any electrical components known in the art for accepting commands and providing an output based on user inputs, pre-programmed data, or combinations thereof. Thus, controller **501** may comprise programmable logic devices, non-field-programmable firmware devices, field programmable firmware devices, microprocessors, microcontrollers, discrete logic and other logic devices adapted to perform the functions of receiving an input from a user and commanding the vibrators or other motors and light sources of the invention to operate in any desired modes, which are further defined herein. Memory **502** may be integral with the controller **501,** such as "on-board memory" or may be one or more separate memory devices in electrical communication with controller **501.** Memory **502** may be utilized to store pre-programmed modes of operation and to store other information as may be required by controller **501.** Battery **503** is in electrical communication with and provides power to drive controller **501,** memory **502,** clitoral light source group **302,** clitoral vibrator **304,** vaginal vibrator **204,** vaginal light source group **202,** and any other electrical devices of the invention. Battery **503** may drive these devices directly or may drive them through their electrical connection with controller **501.** Battery **503** may also be in electrical communication with charge circuit **506,** which may condition power received through charging port connector **451.** Alternatively, charging port connector **451** may be in direct communication with battery **503** for directly charging the battery. Battery **503** may also be in electrical communication with inductive charging circuit **505,** which may be adapted to produce a charging current when placed in proximity with magnetic field, as is known in the art of inductive charging circuits. In a further alternate embodiment, not shown in **Fig. 8****,** battery **503** may comprise a replaceable battery or group of batteries, such that they are replaced with a new battery or group of batteries when a given discharge level is reached, for example, when battery **503** is no longer able to power the improved sexual stimulation device **100** of the invention to a user's satisfaction. In any of the embodiments described herein, battery **503** may be a single battery or a group of batteries. Also, the electrical connection at charging port connector **451** may be any connector suitable to fit within the envelope of the invention and provide electrical communication as described herein. Thus, any type of electrical connector, whether a standard connector or a custom connector, may be used and is therefore within the scope of the claims. In a still further embodiment, charging port connector **451** may be in electrical communication with controller **501** such that modes of operation, firmware for operation of the vibrators and light sources of the invention, programming of keypad button functions, and other programmable aspects of the invention may be download and or uploaded and used by controller **501** and stored in memory **502.** Thus, charging port connector **451** may also be a programming port.

Still referring to **Fig. 8****,** controller **501** may further comprise an infrared or RF wireless interface such as, for example, the interface known as Bluetooth®, the interface known as WiFi, the interface known as Near Field Communications (NFC) or any other wireless interface as is known by persons of ordinary skill in the art, in order to wirelessly control the invention and download new or changed modes of operation information and or other information and the like to be stored in either controller **501** or memory **502,** or the like. Any type of information may be downloaded and stored in this manner. Such wireless interfaces are well known in the electronic arts and, as such, do not require undue experimentation to understand and implement.

Still referring to **Fig. 8****,** controller **501** may be in electrical communication with a vaginal light source group **202** through vaginal finger wires **448** (not shown in **Fig. 8**), a clitoral light source group **302** through clitoral finger wires **443** (not shown in **Fig. 8**), a vaginal vibrator **204** through vaginal finger wires **448** (not shown in **Fig. 8**), and a clitoral vibrator **304** through clitoral finger wires **443.** These devices may be powered off, powered on, pulsed, operated continuously, or driven to various levels of output power directly by the circuitry of controller **501,** or by drivers **452, 453, 454,** or **455** which may be in electrical communication with controller **501,** as the user desires. These drivers may be discrete electric circuits or may be incorporated directly in controller **501** as "on-board" drivers. Vaginal light source group **202,** clitoral light source group **302,** vaginal vibrator **204,** and clitoral vibrator **304** may thus be individually commanded by controller **501** to operate in any patterns desired by the user, which patterns may be coordinated in order to achieve a desired stimulation effect in the user. The patterns of control are discussed below.

Still referring to the preferred embodiment of the invention depicted in **Fig. 8****,** controller **501,** memory **502,** charge circuit **506,** and drivers **452, 453, 454,** and **455** are located on controller printed wiring board **418** (not shown in **Fig. 8**), which may be located in handle **400** (not shown in **Fig. 8**) along with keypad **403.** Battery **503,** charging port connector **451** and inductive charging circuit **505** may also be located in the handle **400** (not shown in **Fig. 8**), and may be connected through discrete wires preferably with solder attachments. Vaginal vibrator **204** and vaginal light source group **202** may be located in vaginal finger **200** (not shown in **Fig. 8**) and clitoral vibrator **304** and clitoral light source group **302** may be located in clitoral finger **300** (not shown in **Fig. 8**).

Exemplary modes of operation and control of the states and patterns used to operate the vibrators and light sources of the invention are now discussed. While a preferred embodiment is discussed for purpose of description herein, it is to be understood that the programmable nature of the invention allows any variation, and therefore any pattern, of intensity and wave shape for both the light sources and vibration of the invention. Thus, the embodiment shown is exemplary only. The intensity of vibration of clitoral vibrator **304** and vaginal vibrator **204** may be controlled by pulse width modulation (PWM) control of power to the vibrator motor by controller **501,** such that, for example, a 50% duty cycle represents LOW power, a 75% duty cycle represents MEDIUM power, and a 100% duty cycle represents HIGH power. These designations of power level may be set as desired, such that various terms may be given to corresponding duty cycles, and any number of power levels, which are the result of variation of the pulse width of the PWM drive signal driving the vibrators, may be defined as desired. It is a feature of controller **501** that any of the vibrators of the invention may be driven at any duty cycle desired, whether constant or in a pattern such as a pulsed pattern, sinusoidal pattern, or otherwise, based upon the desire of the user as programmed into controller **501,** and, preferably, stored into memory **502.**

A user may depress the depressible buttons **405** - **409** on keypad **403** to control operation of the invention in the following manner. It is to be understood that, due to the programmable nature of the invention, any combination of vibration patterns and light patterns may be programmed into controller **501** and memory **502.** The modes of operation described hereinbelow are exemplary and not limiting. In the exemplary embodiment used in for description of operating modes below, vaginal light source group **202** comprises "blue" LEDs, "red" LEDs and an infrared LED; and likewise clitoral light source group **302** comprises "blue" LEDs, "red" LEDs and an infrared LED. Again, these configurations of light source groups are merely exemplary. Furthermore, in the exemplary embodiment used in for description of operating modes below, vaginal finger **200** comprises a vaginal vibrator **204** and clitoral finger 300 comprises clitoral vibrator **304.**

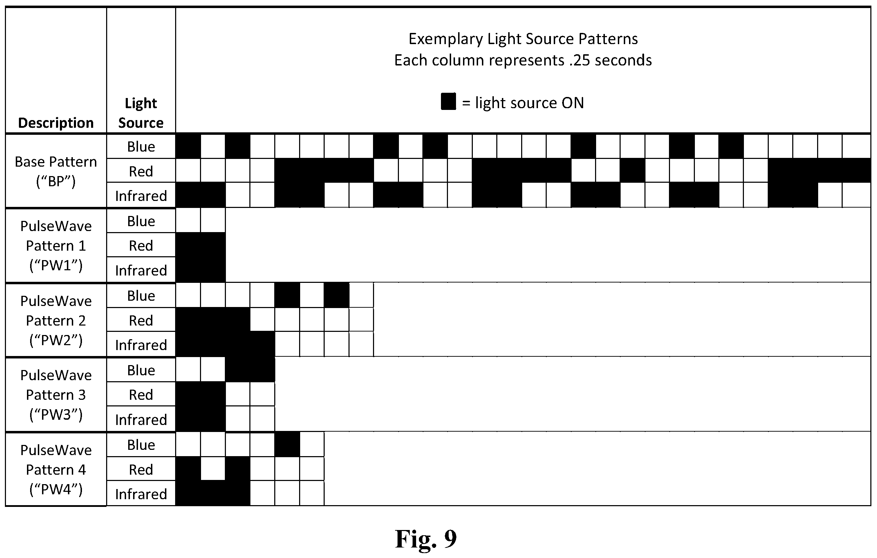

In the exemplary embodiment, the invention may be powered on by pressing first depressible control button **405** and third depressible control button **407** simultaneously for three seconds, which may power the invention ON in a base operating mode. In this mode, vaginal vibrator **204** operates in a continuous LOW power state; the blue, red and infrared LEDs of vaginal light source group **202** are powered in the base pattern depicted in **Fig. 9****;** clitoral vibrator **304** operates in a LOW power state; and the blue, red and infrared LEDs of clitoral light source group **302** are also powered in the base pattern depicted in **Fig. 9****.** Next, pressing and releasing second depressible control button **406** may cause controller **501** to command clitoral vibrator **304** and the light sources of clitoral light source group **302** to an OFF state. Next, pressing and releasing second depressible button **406** may cause controller **501** to command clitoral vibrator **304** to a continuous LOW power state, and cause the light sources of clitoral light source group **302** to operate in the base pattern state of **Fig. 9****,** while commanding vaginal vibrator **204** and the light sources of vaginal light source group **202** to an OFF state. Next, pressing and releasing second depressible button **406** may cause controller **501** to command vaginal vibrator **204** and clitoral vibrator **304** to pulse between OFF and ON states in an out-of-phase pattern in LOW power mode, while commanding the light sources of both vaginal light source group **202** and clitoral light source group **302** to operate in the base pattern shown in **Fig. 9****.** Next, pressing and releasing second depressible button **406** may cause controller **501** to command vaginal vibrator **204** and clitoral vibrator **304** to pulse between OFF and ON states in an in-phase pattern with a high pulse rate, such as, for example, greater than than 2.0 Hz pulse rate, in LOW power mode, while light source groups **202** and **302** remain operating in the base pattern shown in **Fig. 9****.** It is to be understood that the pulse rate may be any rate commanded by controller 501. Next, pressing and releasing second depressible button **406** may cause controller **501** to command vaginal vibrator **204** and clitoral vibrator **304** to operate in a substantially sinusoidal-shaped intensity profile, in which the PWM signal driving each vibrator is characterized by a pulse width that varies sinusoidally. Next, pressing and releasing second depressible button **406** may cause controller **501** to command vaginal vibrator **204** and clitoral vibrator **304,** and light source groups **202** and **302,** back into the base operating mode described above in which vaginal vibrator **204** operates in a continuous LOW power state; the blue, red and infrared LEDs of vaginal light source group **202** are powered in the base pattern depicted in **Fig. 9****;** clitoral vibrator **304** operates in a continuous LOW power state; and the blue, red and infrared LEDs of clitoral light source group **302** are also powered in the base pattern depicted in **Fig. 9****.**

Continuing to discuss now the exemplary mode of operation described above, when the invention is in the base operating mode, pressing third depressible control button **407** alone causes controller **501** to command vaginal vibrator **204** and clitoral vibrator **304** to increase intensity by commanding a higher duty cycle as hereinbefore described, and pressing third depressible control button **407** again causes controller **501** to command vaginal vibrator **204** and clitoral vibrator **304** to further increase intensity. Each press shall result in an increase of 1/6^{th} of intensity range until maximum intensity is reached. Thus, in the exemplary base operating mode described herein, pressing third depressible control button **407** generally results in an increase in perceived vibration intensity to the user. Likewise, when the invention is in the base operating mode, pressing first depressible control button **405** alone causes controller **501** to command vaginal vibrator **204** and clitoral vibrator **304** to decrease intensity by commanding a lower duty cycle. Thus, in the exemplary base operating mode described herein, pressing first depressible control button **405** generally results in a decrease in perceived vibration intensity to the user. In this manner the user may select a desired intensity of vibration. Any number of vibration intensity levels may be programmed into controller **501** and memory **502.**

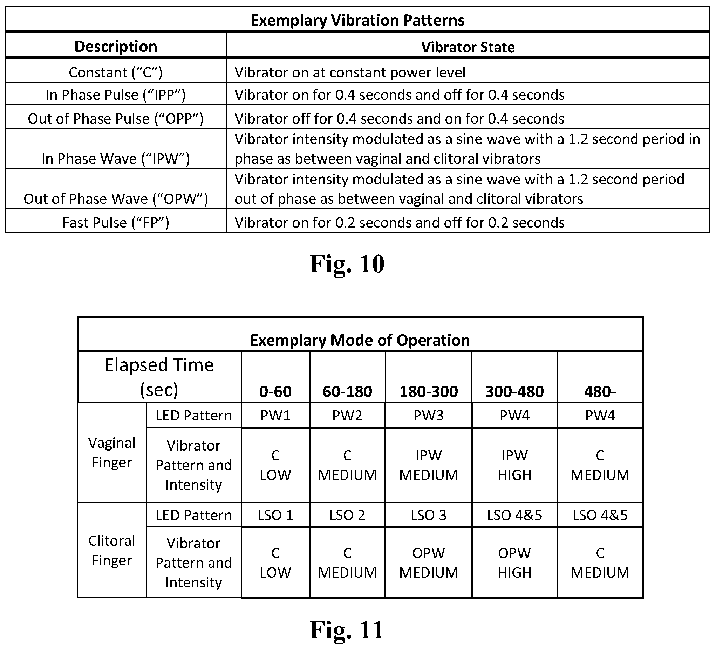

Continuing to discuss now the exemplary mode of operation described above, when the invention is in the base operating mode, depressing and holding fifth depressible control button **409** may cause controller **501** to command vaginal vibrator **204** and clitoral vibrator **304** and the light sources of light source groups **202** and **302** to cycle through a series of programmed states which may include, for the vibrators: continuous ON in LOW power state, continuous OFF state, pulsed ON and OFF in both in-phase and out-of-phase states; and sinusoidal in both in-phase and out-of-phase states; and, for the light source groups **202 and 302:** continuous ON state for all light sources, continuous OFF state for all light sources, the base pattern and other patterns shown in **Fig. 9****.** The invention may cycle through each of these states in series as programmed into controller **501** and memory **502,** taking, for example, approximately seven minutes to complete the cycle. The length of time of the cycle may be programmable, as is the sequencing and description of the states of the vibrators and light sources. Pressing any button during the cycle may return the invention to the base operating mode. A exemplary preferred program of light energy and motor stimulation is shown in Figures 9 and 10.

Continuing to discuss now the exemplary mode of operation described above, when the invention is in the base operating mode, pressing first depressible control button **405** and third depressible control button **407** simultaneously for three seconds may cause controller **501** to command vaginal vibrator **204** and clitoral vibrator **304** to an OFF state, and likewise commend all light sources of vaginal light source group **202** and clitoral light source group **302** to an OFF state.

It is to be noted that pulse width of the PWM drive signal to the vibrators of the invention may be varied independently of one another by controller **501** in any pattern desired such as, for example, continuous ON, continuous OFF, sinusoidal, square, ramped, and so on, by modulation of the pulse width of the PWM drive signal. Also, as herein used, "in-phase" means that the PWM signal to vaginal vibrator **204** and clitoral vibrator **304** are of like waveform with substantially identical timing. "Out-of-phase" means that the PWM signal to vaginal vibrator **204** and clitoral vibrator **304** are of like waveform but are substantially 180 degrees out of phase. It is further to be noted that the PWM drive signals provided to vaginal vibrator **204** and clitoral vibrator **304** are provided independently of one another. Thus, the PWM waveforms to vaginal vibrator **204** and clitoral vibrator **304** may take independently take any shape desired.

Referring now to **Fig. 9****,** and keeping with the exemplary embodiment used in the above description of an exemplary mode of operation of the invention, the timing of various exemplary light source patterns are depicted to provide further detail as to the variety of patterns of light energy emission that may be programmed into controller **501** and memory **502.** It can readily be seen that the pattern defined as Base Pattern, for example, causes the blue and infrared LEDs of a light source group to turn ON for .25 seconds, after which the blue LED turns OFF for .25 seconds while the infrared LED remains ON, and so on. In this manner any number of light source patterns may be defined and programed into controller **501.** Once the patterns have run their course they may repeat. In the examples shown in **Fig. 9****,** five light source patterns are depicted for the exemplary case in which a light source group comprises blue LEDs, red LEDs, and infrared LEDs, in keeping with the exemplary embodiment used in the description of the exemplary mode of operation above. The five exemplary patterns depicted in **Fig. 9** are Base Pattern ("BP"); PulseWave Pattern 1 ("PW1"); PulseWave Pattern 2 ("PW2"); PulseWave Pattern 3 ("PW3"); and PulseWave Pattern 4 ("PW4"). Once such patterns are defined and programmed into controller **501** and memory **502,** they may be used in combination with vibrator patterns also programmed into controller **501** and memory **502** in order to define further exemplary modes of operation of the invention, as discussed below.

Referring next to **Fig. 10****,** and keeping with the exemplary embodiment used in the above description of an exemplary mode of operation of the invention, the timing of various exemplary vibrator patterns are depicted to provide further detail as to the variety of patterns of vibration that may be programmed into controller **501** and memory **502.** It can readily be seen that the pattern defined as Constant, for example, causes the vibrators to operate continuously at a constant power level. In this manner any number of vibration patterns may be defined and programed into controller **501** and memory **502.** Once the patterns have run their course they may repeat. In the examples shown in **Fig. 10****,** six vibration patterns are depicted for the exemplary case in which the invention comprises a vaginal vibrator **204** and a clitoral vibrator **304,** in keeping with the exemplary embodiment used in the description of the exemplary mode of operation above. The six exemplary vibrator patterns depicted in **Fig. 10** are constant ("C"), In Phase Pulse ("IPP"), Out of Phase Pulse ("OPP"), In Phase Wave ("IPW"), Out of Phase Wave ("OPW"), and Fast Pulse ("FP"). Once such patterns are defined and programmed into controller **501** and memory **502,** they may be used in combination with light source patterns also programmed into controller **501** and memory **502** in order to define further exemplary modes of operation of the invention, as discussed below.

Referring now to **Fig. 11****,** another exemplary mode of operation is defined, consistent with the above description of an exemplary mode of operation, for the exemplary embodiment of the invention which both clitoral and vaginal light source groups **302** and **202,** respectively, comprise blue LED light sources, red LED light sources, and infrared LED light sources and in which vaginal finger **200** and clitoral finger **300** comprise a vibrator. It is readily seen from **Fig.11** that in the first sixty seconds, both vaginal vibrator **204** and clitoral vibrator **304** operate in a continuous mode at LOW power while vaginal light source group **202** and clitoral light source group **302** operate in pattern PW1 as was defined in **Fig. 9****.** After the first sixty seconds and until 180 seconds have elapsed, both vaginal vibrator **204** and clitoral vibrator **304** operate in a continuous mode at MEDIUM power while vaginal light source group **202** and clitoral light source group **302** operate in pattern PW2 as was defined in **Fig. 9****.** After 180 seconds have elapsed and until 300 seconds have elapsed, both vaginal vibrator **204** and clitoral vibrator **304** operate in a continuous mode at MEDIUM power while vaginal light source group **202** and clitoral light source group **302** operate in pattern PW3 as was defined in **Fig. 9****.** After 300 seconds have elapsed and until 480 seconds have elapsed, both vaginal vibrator **204** and clitoral vibrator **304** operate in a continuous mode at HIGH power while vaginal light source group **202** and clitoral light source group **302** operate in pattern PW4 as was defined in **Fig. 9****.** After 480 seconds have elapsed, both vaginal vibrator **204** and clitoral vibrator **304** operate in a continuous mode at MEDIUM power while vaginal light source group and clitoral light source group operate in pattern PW4 as was defined in **Fig. 9****.** In this exemplary mode of operation, the invention will continue to run in this state until the user powers the invention to an OFF state as defined above, or presses any button which returns the invention to base motor and light patterns, in an alternate embodiment, the invention may time out after a set period of time.

It is not necessary that the vibrators and light sources of the invention operation in the same pattern at the same time; in fact, the great majority of vibrator and light source patterns may comprise different patterns for the vibrators and light sources of the invention as they are all controlled individually by controller **501.** It is thus readily seen that any pattern of operation of vibration and light source patterns may be programmed into controller **501** and memory **502.**

Although a detailed description of the preferred and alternate embodiments as provided in the description and drawings contains many specifics for the purposes of illustration, a person of ordinary skill in the art will appreciate that many variations and alterations to the details are within the scope of the invention as claimed. Accordingly, the preferred and alternate embodiments of the invention are set forth without any loss of generality to, and without imposing limitations upon, the claimed invention. Thus the scope of the invention should be determined by the appended claims, and not merely by the preferred examples or embodiments given.

### INDUSTRIAL APPLICABILITY

The improved sexual stimulation device using light therapy and vibration of the invention presents a novel and unique structure, not heretofore found in the prior art, which combines vibrational energy and light energy to improve vaginal and clitoral blood flow such that the experience of a user of the invention is enhanced over the devices of the prior art. Additonal benefits may include improved blood supply to genitals and a decrease in both pathogenic bacteria and fungi. The improved sexual stimulation device using light therapy and vibration of the invention has direct applicability to the adult toy industry and related markets, and represents a significant improvement in the state of the art of adult pleasure objects.

## Claims

1. A sexual stimulation device, comprising:
a vaginal finger portion having a first mechanical stimulation means and a first light source group disposed therein, wherein said first light source group is adapted to emit vaginal light energy to enhance genital blood flow from said vaginal finger portion;
a handle portion having a power source, a keypad, and a controller disposed therein;
wherein said vaginal finger portion and said handle portion form a unitary structure configured such that when said vaginal finger portion is slidingly engaged with and inserted into the vagina of a user said handle portion remains outside a user's vagina; and
wherein said controller is in electrical communication with said first mechanical stimulation means, said first light source group, said keypad and said power source, and wherein said controller is adapted to receive power from said power source and to receive commands by user operation of said keypad, and wherein said controller is further adapted to control the operation of said first mechanical stimulation means and said first light source group in response to commands received by a user through said keypad
**characterized in that**
a clitoral finger portion having a second mechanical stimulation means and a second light source group to enhance genital blood flow disposed therein is provided, wherein said second light source group is adapted to emit clitoral light energy from said clitoral finger portion; and
wherein said clitoral finger portion forms a further part of said unitary structure and is configured such that when said vaginal finger portion is slidingly engaged with and inserted into the vagina of a user, said clitoral finger portion engages a user on or near a user's clitoris, while said handle portion remains outside a user's vagina; and
said controller is further defined as being in electrical communication with said second mechanical stimulation means and said second light source group; and wherein said controller is further adapted to control the operation of said second mechanical stimulation means and said second light source group in response to commands received from a user through said keypad.

2. The sexual stimulation device of Claim 1, wherein said first mechanical stimulation means and said second mechanical stimulation means are offset vibrator motors, preferably adapted to operate at a vibration rate of between 5,000 and 25,000 rotations per minute.

3. The sexual stimulation device of Claim 1, wherein said first light source group is oriented such that said vaginal light energy impacts a user's body in the area of the Gräfenberg Spot of a user when said vaginal finger portion is slidingly engaged with and inserted into the vagina of a user and wherein said second light source group is preferably oriented such that said clitoral light energy impacts a user's body in the area of the clitoris of a user when said vaginal finger portion is slidingly engaged with and inserted into the vagina of a user.

4. The sexual stimulation device of Claim 3, wherein said vaginal light energy emitted by said first light source group is between 400nm and 1000nm in wavelength, and wherein said clitoral light energy emitted by said second light source group is also between 400nm and 1000nm in wavelength.

5. The sexual stimulation device of Claim 3, wherein said first light source group is further defined as comprising a first light source emitting light energy with wavelength between 400nm and 515nm, a second light source emitting light energy with wavelength between 610nm and 640nm, and a third light source emitting infrared light energy with wavelength between 820nm to 880nm, and wherein said second light source group is further defined as comprising a fourth light source emitting light energy with wavelength between 400nm and 515nm, a fifth light source emitting light energy with wavelength between 610nm and 640nm, and a sixth light source emitting infrared light energy with wavelength between 820nm and 880nm

6. The sexual stimulation device of Claim 5, wherein said first, second, third, fourth, fifth and sixth light sources are further defined as light emitting diodes, and more specifically said first, second, fourth and fifth light sources are further characterized as having an output power of at least 300 mW with a half-power output angle of +/- 60 degrees; and said third and sixth light sources are further characterized as having output radiant flux of at least 300 millicandelas peak with a half-power output angle of +/- 60 degrees.

7. The sexual stimulation device of Claim 1, further defined as having a memory in communication with said controller, said memory adapted to store pre-programmed modes of operation of said first mechanical stimulation means, said first light sources of said first light source group, said second mechanical stimulation means, and said second light sources of said second light source group said controller is adapted to receive user commands via said keypad selecting a mode of operation from said pre-programmed modes of operation stored in said memory and preferably said memory is further adapted to receive modes of operation downloaded through a programming port.

8. The sexual stimulation device of Claim 7, wherein said programming port is one of an electrical port, a radio frequency wireless port, and an optical wireless port.

9. The sexual stimulation devices of Claim 1, wherein said power source is a rechargeable battery, and wherein the sexual stimulation device further comprises an inductive charging circuit in electrical communication with said rechargeable battery which is adapted to recharge said rechargeable battery by inductive means.

## Patentansprüche

1. Sexualstimulationsgerät, welches aufweist:
einen Vaginalfingerabschnitt, der darin angeordnet eine erste mechanische Stimulationseinrichtung und eine erste Lichtquellengruppe hat, wobei die erste Lichtquellengruppe dazu angepasst ist, vaginale Lichtenergie zur Verbesserung der genitalen Durchblutung aus dem Vaginalfingerabschnitt zu emittieren;
einen Handgriffabschnitt, der eine Stromquelle, eine Tastatur und eine Steuerung darin enthält;
wobei der Vaginalfingerabschnitt und der Handgriffabschnitt eine zusammenhängende Struktur bilden, die derart konfiguriert ist, dass, wenn der Vaginalfingerabschnitt in die Vagina einer Nutzerin eingesetzt und im gleitenden Eingriff mit dieser ist, der Handgriffabschnitt außerhalb der Vagina der Nutzerin verbleibt; und
wobei die Steuerung in elektrischer Verbindung mit der ersten mechanischen Stimulationseinrichtung, der ersten Lichtquellengruppe, der Tastatur und der Stromquelle steht und wobei die Steuerung dazu angepasst ist, Strom von der Stromquelle zu empfangen und Befehle durch eine Nutzertätigung der Tastatur zu empfangen und wobei die Steuerung weiter dazu ausgebildet ist, den Betrieb der ersten mechanischen Stimulationseinrichtung und der ersten Lichtquellengruppe in Reaktion auf Befehle zu steuern, die durch eine Nutzerin über die Tastatur eingegeben wurden,
**dadurch gekennzeichnet, dass** ein Klitorisfingerabschnitt, der eine zweite mechanische Stimulationseinrichtung und eine zweite Lichtquellengruppe zur Verbesserung der genitalen Durchblutung darin angeordnet hat, vorgesehen ist, wobei die zweite Lichtquellengruppe dazu ausgebildet ist, klitorale Lichtenergie aus dem klitoralen Fingerabschnitt zu emittieren, und wobei der klitorale Fingerabschnitt einen weiteren Teil der zusammenhängenden Struktur bildet und derart konfiguriert ist, dass, wenn der Vaginalfingerabschnitt in die Vagina einer Nutzerin eingesetzt ist und im gleitenden Eingriff mit dieser steht, der klitorale Fingerabschnitt die Nutzerin an oder nahe ihrer Klitoris berührt, wobei der Handgriffabschnitt außerhalb der Vagina der Nutzerin verbleibt, und wobei die Steuerung weiter derart definiert ist, dass sie in elektrischer Verbindung mit der zweiten mechanischen Stimulationseinrichtung und der zweiten Lichtquellengruppe steht und wobei die Steuerung weiter dazu ausgebildet ist, den Betrieb der zweiten mechanischen Stimulationseinrichtung und der zweiten Lichtquellengruppe in Reaktion auf Befehle zu steuern, die von der Nutzerin über die Tastatur eingegeben wurden.

2. Sexualstimulationsgerät nach Anspruch 1, wobei die erste mechanische Stimulationseinrichtung und die zweite mechanische Stimulationseinrichtung versetzte Vibratormotoren sind, die bevorzugt dazu ausgebildet sind, bei einer Schwingungsrate zwischen 5.000 und 25.000 min⁻¹ zu arbeiten.

3. Sexualstimulationsgerät nach Anspruch 1, wobei die erste Lichtquellengruppe so orientiert ist, dass die vaginale Lichtenergie den Körper der Nutzerin im Bereich des Gräfenberg-Spots erreicht, wenn der Vaginalfingerabschnitt in die Vagina der Nutzerin eingesetzt und im gleitenden Eingriff mit dieser ist, und wobei die zweite Lichtquellengruppe bevorzugt so orientiert ist, dass die klitorale Lichtenergie auf den Körper der Nutzerin im Bereich der Klitoris der Nutzerin auftrifft, wenn der Vaginalfingerabschnitt in die Vagina der Nutzerin eingesetzt um im gleitenden Eingriff mit dieser ist.

4. Sexualstimulationsgerät nach Anspruch 3, wobei die durch die erste Lichtquellengruppe emittierte vaginale Lichtenergie zwischen 400 nm und 1.000 nm Wellenlänge hat und wobei die durch die zweite Lichtquellengruppe emittierte klitorale Lichtenergie ebenfalls im Bereich zwischen 400 nm und 1.000 nm Wellenlänge liegt.

5. Sexualstimulationsgerät nach Anspruch 3, wobei die erste Lichtquellengruppe weiter so definiert ist, dass sie eine erste Lichtquelle, die Lichtenergie mit einer Wellenlänge zwischen 400 nm und 515 nm emittiert, eine zweite Lichtquelle, die Lichtenergie mit einer Wellenlänge zwischen 610 nm und 640 nm emittiert, und eine dritte Lichtquelle aufweist, die Infrarot-Lichtenergie mit einer Wellenlänge zwischen 820 nm und 880 nm emittiert, und wobei die zweite Lichtquellengruppe weiter so definiert ist, dass sie eine vierte Lichtquelle, die Lichtenergie mit einer Wellenlänge zwischen 400 und 515 nm, eine fünfte Lichtquelle emittiert, die Lichtenergie mit einer Wellenlänge zwischen 610 nm und 640 nm emittiert, und eine sechste Lichtquelle aufweist, die Infrarot-Lichtenergie mit einer Wellenlänge zwischen 820 nm und 880 nm emittiert.

6. Sexualstimulationsgerät nach Anspruch 5, wobei die erste, zweite, dritte, vierte, fünfte und sechste Lichtquelle weiter so definiert sind, dass sie LEDs aufweisen und spezieller dass die erste, zweite, vierte und fünfte Lichtquelle weiter **dadurch gekennzeichnet sind, dass** sie eine Ausgangsleistung von mindestens 300 mW mit einem Halbleistungs-Austrittswinkel von +/- 60 Grad haben und wobei die dritte und sechste Lichtquelle weiter **dadurch gekennzeichnet sind, dass** sie einen Ausgangs-Strahlungsfluss von mindestens 300 Millicandela Spitze und einen Halbleistungs-Austrittswinkel von +/- 60 Grad haben.

7. Sexualstimulationsgerät nach Anspruch 1, weiter so definiert, dass es einen Speicher in Kommunikation mit der Steuerung hat, wobei der Speicher dazu ausgebildet ist, vorprogrammierte Betriebsmodi der ersten mechanischen Stimulationseinrichtung, der ersten Lichtquellen der ersten Lichtquellengruppe, der zweiten Stimulationseinrichtung und der zweiten Lichtquellen der zweiten Lichtquellengruppe zu speichern, und wobei die Steuerung dazu ausgebildet ist, Nutzerbefehle über die Tastatur zu empfangen, die einen Betriebsmodus von den vorprogrammierten Betriebsmodi auszuwählen, die in dem Speicher gespeichert sind, wobei bevorzugt der Speicher weiter dazu ausgebildet ist, Betriebsmodi zu empfangen, die über einen Programmieranschluss heruntergeladen werden.

8. Sexualstimulationsgerät nach Anspruch 7, wobei der Programmieranschluss eines von einem elektrischen Anschluss, einem drahtlosen HF-Anschluss und einem drahtlosen optischen Anschluss ist.

9. Sexualstimulationsgerät nach Anspruch 1, wobei die Stromquelle eine aufladbare Batterie ist und wobei die Sexualstimulationseinrichtung weiter eine Induktions-Aufladeschaltung in elektrischer Verbindung mit der aufladbaren Batterie aufweist, welche dazu ausgebildet ist, die aufladbare Batterie durch Induktionsmittel aufzuladen.

## Revendications

1. Dispositif de stimulation sexuelle, comprenant :
une partie doigt vaginal présentant un premier moyen de stimulation mécanique et un premier groupe de sources lumineuses disposés dans celle-ci, sachant que ledit premier groupe de sources lumineuses est apte à émettre de l'énergie lumineuse vaginale pour augmenter une circulation sanguine génitale depuis ladite partie doigt vaginal ;
une partie poignée présentant une source de puissance, un pavé de touches, et une unité de commande disposés dans celle-ci ;
sachant que ladite partie doigt vaginal et ladite partie poignée forment une structure unitaire configurée de telle sorte que lorsque ladite partie doigt vaginal est en prise de manière coulissante avec et insérée dans le vagin d'une utilisatrice, ladite partie poignée reste en dehors du vagin d'une utilisatrice ; et
sachant que ladite unité de commande est en communication électrique avec ledit premier moyen de stimulation mécanique, ledit premier groupe de sources lumineuses, ledit pavé de touches et ladite source de puissance, et sachant que ladite unité de commande est apte à recevoir de la puissance depuis ladite source de puissance et à recevoir des consignes suite à un fonctionnement dudit pavé de touches par une utilisatrice, et sachant que ladite unité de commande est en outre apte à commander le fonctionnement dudit premier moyen de stimulation mécanique et dudit premier groupe de sources lumineuses en réponse à des consignes reçues d'une utilisatrice via ledit pavé de touches,
**caractérisé en ce que**
une partie doigt clitoridien présentant un deuxième moyen de stimulation mécanique et un deuxième groupe de sources lumineuses pour augmenter une circulation sanguine génitale disposés dans celle-ci est prévue, sachant que ledit deuxième groupe de sources lumineuses est apte à émettre de l'énergie lumineuse clitoridienne depuis ladite partie doigt clitoridien ; et
sachant que ladite partie doigt clitoridien forme une partie supplémentaire de ladite structure unitaire et est configurée de telle sorte que lorsque ladite partie doigt vaginal est en prise de manière coulissante avec et insérée dans le vagin d'une utilisatrice, ladite partie doigt clitoridien soit en prise avec une utilisatrice sur ou près du clitoris d'une utilisatrice, tandis que ladite partie poignée reste à l'extérieur du vagin d'une utilisatrice ; et
ladite unité de commande est en outre définie comme étant en communication électrique avec ledit deuxième moyen de stimulation mécanique et ledit deuxième groupe de sources lumineuses ;
et sachant que ladite unité de commande est en outre apte à commander le fonctionnement dudit deuxième moyen de stimulation mécanique et dudit deuxième groupe de sources lumineuses en réponse à des consignes reçues d'une utilisatrice via ledit pavé de touches.

2. Le dispositif de stimulation sexuelle de la revendication 1, sachant que ledit premier moyen de stimulation mécanique et ledit deuxième moyen de stimulation mécanique sont des moteurs vibratoires décalés, de préférence aptes à fonctionner à une cadence de vibration comprise entre 5 000 et 25 000 rotations par minute.

3. Le dispositif de stimulation sexuelle de la revendication 1, sachant que ledit premier groupe de sources lumineuses est orienté de telle sorte que ladite énergie lumineuse vaginale rencontre le corps d'une utilisatrice dans la zone du point de Gräfenberg d'une utilisatrice lorsque ladite partie doigt vaginal est en prise de façon coulissante avec et insérée dans le vagin d'une utilisatrice et sachant que ledit deuxième groupe de sources lumineuses est de préférence orienté de telle sorte que ladite énergie lumineuse clitoridienne rencontre le corps d'une utilisatrice dans la zone du clitoris d'une utilisatrice lorsque ladite partie doigt vaginal est en prise de façon coulissante avec et insérée dans le vagin d'une utilisatrice.

4. Le dispositif de stimulation sexuelle de la revendication 3, sachant que ladite énergie lumineuse vaginale émise par ledit premier groupe de sources lumineuses est comprise entre 400 nm et 1000 nm de longueur d'onde, et sachant que ladite énergie lumineuse clitoridienne émise par ledit deuxième groupe de sources lumineuses est également compris entre 400 nm et 1000 nm de longueur d'onde.

5. Le dispositif de stimulation sexuelle de la revendication 3, sachant que ledit premier groupe de sources lumineuses est en outre défini comme comprenant une première source lumineuse émettant de l'énergie lumineuse d'une longueur d'onde comprise entre 400 nm et 515 nm, une deuxième source lumineuse émettant de l'énergie lumineuse d'une longueur d'onde comprise entre 610 nm et 640 nm, et une troisième source lumineuse émettant de l'énergie lumineuse infrarouge d'une longueur d'onde comprise entre 820 nm et 880 nm, et
sachant que ledit deuxième groupe de sources lumineuses est en outre défini comme comprenant une quatrième source lumineuse émettant de l'énergie lumineuse d'une longueur d'onde comprise entre 400 nm et 515 nm, une cinquième source lumineuse émettant de l'énergie lumineuse d'une longueur d'onde comprise entre 610 nm et 640 nm, et une sixième source lumineuse émettant de l'énergie lumineuse infrarouge d'une longueur d'onde comprise entre 820 nm et 880 nm.

6. Le dispositif de stimulation sexuelle de la revendication 5, sachant que lesdites première, deuxième, troisième, quatrième, cinquième et sixième sources lumineuses sont en outre définies comme diodes électroluminescentes, et plus spécifiquement lesdites première, deuxième, quatrième et cinquième sources lumineuses sont en outre caractérisées comme ayant une puissance de sortie d'au moins 300 millicandelas avec un angle de sortie à mi-puissance de +/- 60 degrés ; et lesdites troisième et sixième sources lumineuses sont en outre caractérisées comme ayant un flux de radiation de puissance d'au moins 300 mW de crête avec un angle de sortie à mi-puissance de +/- 60 degrés.

7. Le dispositif de stimulation sexuelle de la revendication 1, défini en outre comme ayant une mémoire en communication avec ladite unité de commande, ladite mémoire étant apte à mémoriser des modes de fonctionnement préprogrammés dudit premier moyen de stimulation mécanique, desdites premières sources lumineuses dudit premier groupe de sources lumineuses, dudit deuxième moyen de stimulation mécanique, et desdites deuxièmes sources lumineuses dudit deuxième groupe de sources lumineuses, ladite unité de commande est apte à recevoir des consignes d'une utilisatrice via ledit pavé de touches sélectionnant un mode de fonctionnement parmi lesdits modes de fonctionnement préprogrammés mémorisés dans ladite mémoire et de préférence ladite mémoire est en outre apte à recevoir des modes de fonctionnement téléchargés via un port de programmation.

8. Le dispositif de stimulation sexuelle de la revendication 7, sachant que ledit port de programmation est l'un d'un port électrique, d'un port radiofréquence sans fil, et d'un port optique sans fil.

9. Le dispositif de stimulation sexuelle de la revendication 1, sachant que ladite source de puissance est une pile rechargeable, et sachant que le dispositif de stimulation sexuelle comprend en outre un circuit de charge inductif en communication électrique avec ladite pile rechargeable, qui est apte à recharger ladite pile rechargeable par induction.
